(19)

Europäisches
Patentamt

European
Patent Office

Office européen
des brevets

(11)　EP 2 276 881 B1

(12)　EUROPEAN PATENT SPECIFICATION

(45) Date of publication and mention
of the grant of the patent:
**29.08.2018  Bulletin 2018/35**

(21) Application number: **09733511.1**

(22) Date of filing: **03.04.2009**

(51) Int Cl.:
**D04B 21/12** (2006.01)　　　**D06N 3/14** (2006.01)

(86) International application number:
**PCT/IB2009/005389**

(87) International publication number:
**WO 2009/127966 (22.10.2009 Gazette 2009/43)**

(54) **ELASTIC KNIT FABRICS WITH CROSS DIRECTION STRETCH**

ELASTISCHE STRICKSTOFFE MIT QUERRICHTUNGSDEHNUNG

TRICOTS ÉLASTIQUES À ÉTIREMENT DANS LE SENS TRANSVERSAL

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **14.04.2008  US 44640 P**

(43) Date of publication of application:
**26.01.2011  Bulletin 2011/04**

(73) Proprietor: **Invista Technologies S.à.r.l.
9000 St. Gallen (CH)**

(72) Inventors:
• **TERRANEO, Leopoldo
I-34072 Gradisco d'Isonzo (IT)**

• **PIERMATTEI, Alessandro
I-34071 Cormons (IT)**

(74) Representative: **Cockerton, Bruce Roger
Carpmaels & Ransford LLP
One Southampton Row
London WC1B 5HA (GB)**

(56) References cited:
**WO-A-83/01736　　　WO-A-97/37073
GB-A- 1 581 295　　　US-A- 3 892 425**

Remarks:
The file contains technical information submitted after
the application was filed and not included in this
specification

**Description**

**FIELD OF THE INVENTION**

The present invention relates to the preparation of stretchable elastic knit fabrics. Such fabrics are consolidated warp knit fabrics which have been impregnated with elastomeric polymer.

**BACKGROUND OF THE INVENTION**

Existing elastic fabrics can be produced by well known knitting or weaving processes using elastic fibers such as spandex or elastane. Typical processes involving the use of elastic fiber materials to produce knit fabrics are described, for example, in U.S. Patent No. 4,103,485 and in PCT Patent Application Nos. WO 00/29654 and WO 04/022827. Knit fabrics prepared from elastomeric yarns and fibers can be expensive because of the high cost of such fibers and yarns. Knit fabrics of this type also may require expensive scouring and dying processes to color the fabrics into a final form.

Impregnation of various types of fabrics with elastomeric material to impart or improve elasticity and stretch of such fabric is also well known. U.S. Patent No. 4,366,814, for example, discloses impregnation of elastomer into extensible woven, knit or nonwoven fabric substrates in order to provide elastic bandage products.

Alteration of fabric stretch characteristics can also be provided by combining elastomeric materials with fabric substrates which have been drawn, consolidated or "necked". U.S. Patent No. 5,910,224 and 6,942,896, for example, both disclose the use of elastomeric materials applied to neckable or necked nonwoven substrates to provide extensible fabric products for various uses.

WO 83/01736 discloses a bandage material for forming in place an orthopedic cast comprising a pliant, large mesh fabric carrier formed from a knit of a high-strength, high-modulus yarn with a low moisture pick-up, the fabric carrier having openings of relatively large transverse dimension, and being coated with a polymer composition comprising a cold water curable polyurethane prepolymer comprising the reaction product of a polyalkylene ether diol, a polyalkylene ether triol and a diisocyanate.

WO 97/37073 discloses a composite sheet suited for use as artificial leather prepared by treating with an elastomeric resin a fabric having an interior yarn network and an outer layer of well separated fibers that loop in and out of the yarn network. The resin completely or partially impregnates the outer layer

Notwithstanding the availability of a wide variety of stretchable fabrics comprising the combination of elastomeric materials with various kinds of fabric substrates, it would be advantageous to identify additional types of elastic fabrics which are of relatively low cost and which have desirable stretch properties, especially in the cross-direction.

**SUMMARY OF THE INVENTION**

The present invention is defined by the claims. The present disclosure is directed to an elastic fabric with cross-direction stretch. Such an elastic fabric comprises a warp knit fabric which has an initial basis weight of from about 15 to 50 grams per square meter and which has been drawn to the extent that it has been consolidated to between about 30% and 90% of its initial width. This consolidated warp knit fabric is then substantially uniformly impregnated with an elastomeric polymer to the extent that the impregnated fabric exhibits cross direction elongation greater than about 50%. Such elastic fabric can be incorporated into a variety of articles such as articles of apparel or medical or personal hygiene articles.

The present invention is directed to a preferred method for forming elastic knit fabrics. Such a preferred method comprises as a first step providing a warp knit fabric having an initial width, a thickness, first and second outer surfaces, a machine direction, a cross- direction, and an initial basis weight of from 15 to 50 grams per square meter. In a second step, this warp knit fabric is then uniformly drawn by applying a machine direction force to thereby provide a consolidated warp knit fabric having a cross-direction width which is from 30% to 90% of its initial width. In a third method step, this consolidated warp knit fabric is substantially uniformly impregnated with a solution comprising an elastomeric polymer dissolved in a solvent. In a final method step, solvent is removed from the impregnated consolidated warp knit fabric by wet coagulation to thereby deposit the elastomeric polymer substantially uniformly throughout the thickness of the consolidated warp knit fabric.

**DETAILED DESCRIPTION OF THE INVENTION**

The elastic fabrics of the present disclosure are prepared from warp knit fabrics which can be consolidated by drawing and which are then impregnated with elastomeric polymer. Warp knit fabrics are well known materials. They can be conventionally produced on Ketten, Raschel or tricot knitting machines. Processes and apparatus for preparing warp knit fabrics are disclosed, for example, in U.S. Patent Nos. 4,487,040; 4,802,346 and 6,122,940 and in PCT Patent Application Nos. WO 88/020038 and WO 03/023105.

The yarns used in the warp knit fabrics of this disclosure can be formed from continuous or discontinuous fibers. These yarn fibers may be made, for example, of polyester, e.g., polyethylene terephthalate, or of polyamide, e.g., nylon. The fibers used in the warp knit fabrics herein will generally be inelastic.

The warp knit fabrics used to prepare the elastic fabrics herein will generally have an initial basis weight of from 15 to 50 grams per square meter. More preferably, the initial basis weight of the warp knit fabrics used herein will range from about 20 to 40 grams per square meter. The term "initial basis weight" as used herein refers to the basis weight of the warp knit fabrics before they are consolidated and impregnated with elastomeric polymer.

To prepare the elastic fabrics herein, these warp knit fabrics are first uniformly drawn by the application of machine direction force in order to produce consolidated warp knit fabrics. Such consolidated warp knit fabrics will have a width, i.e., dimension in the cross-direction, which ranges from 30% to 90%, of the initial width of the warp knit fabric prior to consolidation. More preferably, the warp knit fabrics are consolidated to the extent of having a cross-direction dimension which ranges from about 50% to 70% of the initial width of the fabric.

The warp knit fabric material, which has been consolidated to the requisite extent is then substantially uniformly impregnated with an elastomeric polymer to produce the elastic fabrics of this disclosure. The term "elastomeric polymer" as used herein refers to any polymer that, when formed into a sheet, fiber, or film and upon application of a biasing force, is elongatable to a stretched length that is at least about 160 percent of its relaxed unbiased length and that will recover at least 55 percent of its elongation upon release of the elongating biasing force. For example, a one centimeter sample of material that is elongatable to at least 1.6 centimeters and which, upon being elongated to 1.6 centimeters by application of a force and with release of the force, will recover to a length of not more than 1.27 centimeters. Many elastomeric materials exist which may be stretched by much more than 60% of their relaxed length, for example 100 percent or more, and many of these will recover to substantially their original relaxed length, for example, to within 105 percent of their original relaxed length, upon release of the stretching force.

Elastomeric polymers useful in this invention include polyurethanes, styrene-butadiene block copolymers, and polyether-ester block copolymers. In a preferred embodiment, the elastomeric polymer is a polyurethane. In a most preferred embodiment, the polyurethane will be Lycra® spandex.

Elastomeric polyurethanes useful in this invention can be prepared by reacting a polymeric glycol with a diisocyanate to form a capped glycol, dissolving the capped glycol (in a suitable solvent), and then reacting the capped glycol with a difunctional chain extender having active hydrogen atoms. Such polyurethanes are termed "segmented" because they are comprised of "hard" urethane and urea segments derived from the diisocyanate and chain extender and "soft" segments derived primarily from the polymeric glycol. Suitable solvents for preparing solutions of such polymers are amide solvents such as dimethylacetamide ("DMAc"), dimethylformamide ("DMF"), and N-methyl-pyrrolidone, but other solvents such as dimethylsulfoxide and tetramethylurea can also be used.

Polymeric glycols used in the preparation of the elastomeric polyurethanes include polyether glycols, polyester glycols, polycarbonate glycols and copolymers thereof. Examples of such glycols include poly(ethyleneether) glycol, poly(tetramethyleneether) glycol, poly(tetramethylene-co-2-methyl-tetramethyleneether) glycol, poly(ethylene-co-butylene adipate) glycol, poly(2,2-dimethyl-1,3-propylene dodecanoate) glycol, poly(pentane-1,5-carbonate) glycol, and poly(hexane-1,6-carbonate) glycol.

Useful diisocyanates include 1-isocyanato-4-[(4-isocyanatophenyl)methyl]benzene, 1-isocyanato-2-[(4-isocyanatophenyl)methyl]benzene, isophorone diisocyanate, 1,6-hexanediisocyanate, and 2,4-tolylene diisocyanate.

The chain extender can be a diol or a diamine. Useful diols include ethylene glycol, 1,3-trimethylene glycol, 1,4-butanediol, and mixtures thereof. Use of diol chain extenders leads to polyurethanes. Useful diamines include ethylene diamine, 1,2-propanediamine, 2-methyl-1,5-pentanediamine, 1,3-diaminopentane, 1,4-cyclohexane-diamine, 1,3-cyclohexanediamine, and mixtures thereof. In this case, the polymer produced is a polyurethaneurea (a sub-class of polyurethanes). When a polyether glycol and a diamine chain extender are utilized, the polymer produced is a polyetherurethaneurea; when a polyester glycol is utilized in combination with a diamine chain extender, a polyesterurethaneurea is produced. Monofunctional amine chain terminators such as diethyl amine, butylamine, cyclohexylamine, and the like can be added to control the molecular weight of the polymer. In a preferred embodiment, the elastomeric polymer is a diamine-extended polyurethane elastomer.

The elastomeric polymer will generally be impregnated into the consolidated warp knit fabrics herein in the form of a solution containing the elastomeric polymer and a solvent. Solvents suitable for preparing the elastomeric polymer solutions include dimethylacetamide, dimethylformamide, and N-methyl-pyrrolidone. The viscosity of the elastomeric polymer solution is directly related to the concentration of the polymeric material in solution and consequently, the solution viscosity can influence both the degree of penetration of the polymer into the consolidated warp knit fabric and the amount of polymer deposited therein. When the solution viscosity is too low, insufficient amounts of elastomer may be deposited in the consolidated warp knit material resulting in poor elasticity characteristics. When the solution viscosity is too high, penetration of the solution into the consolidated warp knit fabric may be reduced, thereby resulting in incomplete or nonuniform impregnation of the polymer into the fabric or formation of a layer of the polymer on the surface of the fabric.

The solution of elastomeric polymer to be impregnated into the consolidated warp knit fabric suitably has a solution viscosity to provide adequate flowability and sufficient delivery of, for example, polyurethaneurea at application temperature, pressure and other process conditions. In one embodiment, for example, the solution can have a solution viscosity of approximately 10,700 centipoise ("cPs"), typically 10,000 - 40,000 cPs, measured at 75 degrees centigrade. In one embodiment, the solution can comprise from about 5 wt% to about 20 wt% polymer.

In one embodiment, the consolidated knit fabric at least partially sorbs the polymer solution. In other embodiments, the consolidated knit fabric may adsorb the polymer solution. And in still other embodiments, the consolidated knit fabric may absorb the polymer solution. In another embodiment, the polymer solution substantially uniformly impregnates the fabric. The term "substantially uniformly impregnates" as used herein means that the concentration of the polymer at a second surface of the consolidated warp knit fabric is within 25% of the concentration of the polymer at a first surface of the consolidated warp knit fabric. The consolidated warp knit fabric should therefore not be coated or otherwise treated in such a way as to prevent the polymer solution from being absorbed into the fabric. The elastomeric polymer solution and/or the knit fabric can include a surface-active agent to facilitate the impregnation of the fabric by the polymeric solution. Suitable surface-active agents include non-ionic wetting agents such as polymeric surfactants.

Additives, for example, pigments, antioxidants, ultraviolet light stabilizers, antimicrobial agents and lubricants, can be added in small quantities to the elastomeric polymer solution, provided such additives do not detract from the benefits of the invention.

Any suitable method of coating the elastomeric polymer solution onto the consolidated warp knit fabric or otherwise impregnating the fabric can be used as long as the fabric is uniformly impregnated and the coating is not concentrated on one or the other surface of the fabric. It should be noted that, although coating methods may be employed to treat the consolidated warp knit fabric with the elastomeric polymer solution, the solution and fabric properties and the coating process conditions are selected such that the polymer solution completely wets the consolidated warp knit fabric or is otherwise completely absorbed into or driven into the consolidated warp knit fabric so that a polymeric layer is not formed on either surface of the fabric. In general, the amount of polymeric solution applied during coating can be controlled by utilizing a coating implement held at a predetermined distance above the fabric.

The solution can also be mechanically pressed into the fabric. Rollers, platens, scrapers, knives, and the like can be used in the process of this invention as coating implements. Spraying the solution onto the consolidated warp knit fabric can also be effective provided that the elastomeric solution substantially completely and uniformly impregnates the knit fabric. The force of the spray can be adjusted to assist in obtaining good penetration. The consolidated warp knit fabric may be impregnated with the elastomeric polymer solution using a process known in the art as a "dip and squeeze" method in which the knit fabric is dipped or otherwise immersed into a tank containing the elastomeric polymer solution followed by squeezing, such as between nip rolls, to remove excess polymer solution. This method is preferred in order to minimize differences between the two surfaces of the stretchable elastic fabric prepared.

The consolidated warp knit fabric can be impregnated with sufficient polymer solution to provide the desired amount of elastomeric polymer in the final impregnated nonwoven sheet. The consolidated warp knit fabric can be impregnated with sufficient polymer solution to deposit therein between about 15 and about 55 weight percent elastomeric polymer, for example between about 20 and about 40 weight percent of elastomeric polymer, based on the total weight of elastomeric polymer and consolidated warp knit fabric.

Once the consolidated warp knit fabric has been impregnated with a solution comprising a solvent and an elastomeric polymer, the solvent is removed. The solvent is preferably removed by wet coagulation followed by removal of the coagulating liquid. The term "wet coagulation" is used herein to describe a process in which a consolidated warp knit fabric, having impregnated therein a solution comprising an elastomeric polymer dissolved in a solvent, is contacted with a coagulating liquid which is a non-solvent for the elastomeric polymer but is miscible with the solvent used to form the elastomeric polymer solution. The coagulating liquid is also selected such that it does not dissolve the consolidated warp knit fabric. The coagulating liquid causes the polymeric material to be coagulated and the solvent to be removed into the coagulating liquid. The coagulating liquid is subsequently removed from the polymer-impregnated consolidated warp knit fabric, such as by air drying or heating.

Wet coagulation provides a product having a surprisingly softer, more cloth-like hand than thermal drying. Wet coagulation processes are well known in the art and are commonly used in the production of artificial leathers. Water is preferred as the coagulating liquid due to ease of handling and low cost. Other suitable coagulating liquids include methanol, ethanol, isopropanol, acetone, or methylethyl ketone. A solvent for the elastomeric polymer such as dimethylformamide, dimethylacetamide, or N-methyl-pyrrolidone or other additives such as surfactants may be added to the coagulating liquid to modify the rate of coagulation. In addition, the temperature of the coagulation bath can be controlled to change the coagulation rate. Slower coagulation rates give the impregnated consolidated warp knit fabric a more attractive hand after the solvent has been removed.

The hand of the consolidated warp knit fabric can be improved by sanding or napping to raise fibers on the surface of the fabric prior to elastomer impregnation. Napping involves passing a fabric over a rotating roll that contains small metal points that effectively brush the fabric to raise fibers to the surface. In sanding, the metal brush is replaced with a rotating roll coated with sandpaper. Typically, the consolidated warp knit fabric can be napped or sanded on both surfaces. For example, the fabric can be sanded with 80 to 200 grit sandpaper.

The elastic fabrics prepared in accordance with the present invention will generally exhibit a percent elongation in the machine direction of less than about 5%. The percent elongation in the cross direction of such fabric will generally be greater than 50%, more preferably between about 100% and 300%.

The stretchable elastic fabrics of the present disclosure are useful as elastic interliners for various garments, particularly in jackets and coats. Interliners are fabrics inserted between the outer and inner layers of a garment that are intended to impart or to improve shape retention, padding, insulating value, stiffening, or bulk to a garment. The elastic fabrics herein are particularly useful for this application because of their low cost combined with permanent elasticity and the ability to provide stretch for comfort in the around-the-body dimension.

Stretchable elastic fabrics falling within the scope of the present disclosure can be provided so as to be highly comfortable to body contours and can serve a therapeutic purpose by applying elastically-resilient pressure over an injured or wounded area. Accordingly, the elastic fabrics herein can be useful in medical articles and applications. Such articles and applications may, for example, include: tapes, including adhesive and cohesive tapes; wraps, including compression wraps; bandages; dressings, including surgical and wound dressings; and surgical drapes, including incise drapes that are adhered to the skin surrounding a surgical incision. The elastic fabrics herein can also be used in personal hygiene articles such as diapers and other incontinence control or catamenial articles.

TEST METHODS

Various tests and procedures can be used to determine a number of properties and characteristics of the stretchable elastic fabrics of this disclosure. Some of the more typical tests used to evaluate fabrics of the type disclosed herein are summarized as follows:

Force Required to Elongate to 30%

This analysis is performed on an Instron Model 5565 equipped with the Merlin data collection software system. Both the Merlin system and instrument hardware are available from Instron Corporation (Braintree, Massachusetts). A one inch +/- 0.05 inch wide (2.54 cm +/- 0.13 cm) and approximately 8 inch (20.32 cm) long sample of an elastic fabric is clamped in the jaws of the Instron machine with a sample length set at 3.00 inches (7.62 cm). The sample is prepared such that the length of the sample is aligned with the cross-direction of the fabric. The sample is elongated at a rate of six inches per minute (15.24 cm/min) to an elongation of 30%. The force in grams at 50% elongation is recorded.

Break Tenacity Analysis

This analysis is performed on an Instron Model 5565 equipped with the Merlin data collection software system. Both the Merlin system and instrument hardware are available from Instron Corporation (Braintree, Massachusetts). A one inch +/- 0.05 inch wide (2.54 cm +/-0.13 cm) and approximately 8 inch (20.32 cm) long sample of an elastic fabric sheet is clamped in the jaws of the Instron machine with a sample length set at 3.00 inches (7.62 cm). The sample is prepared such that the length of the sample is aligned with the cross-direction of the fabric. The sample is elongated at a rate of six inches per minute (15.24 cm/min) until the sample breaks into two portions and the maximum force in grams at the break point is recorded.

Basis Weight

A rectangular sample of elastic fabric or elastic fabric precursor approximately 1.0 inch by 8.0 inches (2.54 cm by 20.32 cm) is relaxed with care so that the sample contains no puckers or wrinkles. The length and width of the sample are measured to the nearest millimeter, and the sample is weighed to the nearest tenth of a milligram. The weight is divided by the calculated area and the result expressed in terms of grams per square meter to the nearest 0.1 gram.

Load and Unload Force Analysis

This analysis is performed on an Instron Model 5565 equipped with the Merlin data collection software system. Both the Merlin system and instrument hardware are available from Instron Corporation (Braintree, Massachusetts). A one inch +/- 0.05 inch wide (2.54 cm +/-0.13 cm) and approximately 8 inch (20.32 cm) long sample of an elastic fabric sheet is clamped in the jaws of the Instron machine with a sample length set at 3.00 inches (7.62 cm). The sample is prepared such that the length of the sample is aligned with the cross-direction of the nonwoven. The sample is elongated at a rate of six inches per minute (15.24 cm/min) to an elongation of 140% and then relaxed to its original length. This is repeated two more times and on the third cycle the force exerted by the material on the extension cycle (Load Force) is recorded at 50%, 100% and 135% elongation based on the original sample length and similarly, the force exerted by the material on the third relaxation cycle (Unload Force) is also recorded at the same elongation points. Results are expressed as Third Cycle Load and Unload forces, in grams, at the appropriate percent elongation.

Percent Elongation Analysis

A relaxed strip of elastic fabric 1.0 inch (2.54 cm) wide and approximately 8 inches (20.32 cm) long that is free of puckers or wrinkles is marked with a pen at two points 4.0 inches (10.2 cm) apart such that the marks are approximately equal distance from the ends of the fabric. The ends of the fabric are then firmly held by the thumb and forefinger of each hand and the sample is fully extended, but not extended so far that the sample is torn or suffers any similar mechanical damage. The point of maximum elongation is apparent to the person performing the test as a noticeable increase in resistance to extension by the fabric. The length between the two marked points on the nonwoven is then measured and the percent elongation calculated by the following formula, where the initial length is 10.2 cm:

$$\text{Percent Elongation} = \{(\text{elongated length} - \text{initial length}) / \text{initial length}\} \times 100\%$$

When the percent elongation is measured in the consolidated direction, the fabric sample is cut with the length aligned with the cross-direction (consolidated direction).

## EXAMPLES

Several samples of warp knit fabrics made of either polyethylene terephthalate (PET) or polyamide (PA) i.e., nylon, are fed through a nip roll operating at a first speed and then to a second nip roll operating at a second speed faster than that of the first nip roll and then onto a take up roll. In this process, the 170 cm wide warp knit fabrics are consolidated to a final width of 110 cm. The several consolidated warp knit fabric samples have essentially zero machine direction elongation. The initial pre-consolidation basis weights of these samples are shown in Table 1 below.

These consolidated warp knit fabric samples are then uniformly impregnated with a 15% solution of LYCRA spandex elastomer in a dimethylformamide (DMF) solvent.

The impregnated consolidated warp knit fabric samples are then immersed in a 70°F (21°C) bath of 25% by volume DMF in water. After about one minute, the impregnated fabrics are successively transferred to a series of water wash tanks, with each tank in the series containing a decreased concentration of DMF such that the last wash tank in the series contains about 100% water. The impregnated fabric samples are then dried in air at room temperature.

The elastic warp knit fabric samples so prepared have final basis weight and elongation values as shown in Table I.

Table I

| Example No. | Knit Fiber Type | Initial Basis Wt. (g/m$^2$) | Final Basis Wt (g/m$^2$) | % Elongation @ 500g | % Elongation @ 1000g |
|---|---|---|---|---|---|
| I | PET | 37 | 87.8 | 122 | 278 |
| II | PET | 35 | 84.5 | 88 | 128 |
| III | PET | 35 | 86.6 | 96 | 154 |
| IV | PET | 25 | 68.1 | 234 | 313 |
| V | PET | 25 | 61.6 | 258 | 275 |
| VI | PA | 25 | 55.6 | 272 | 308 |
| VII | PA | 25 | 57.5 | 234 | 302 |
| VIII | PA | 35 | 96.8 | 82 | 198 |

**Claims**

1.  A method for forming a stretchable elastic fabric, which method comprises the steps of:

    a) providing a warp knit fabric having a thickness, an initial width, first and second outer surfaces, a machine direction, a cross-direction, and an initial basis weight of from 15 to 50 grams per square meter;
    b) uniformly drawing said warp knit fabric by applying a machine direction force to thereby provide a consolidated warp knit fabric having a cross-direction width which is from 30% to 90% of its initial width;
    c) substantially uniformly impregnating said consolidated warp knit fabric with a solution comprising an elastomeric polymer dissolved in a solvent; and
    d) removing solvent from the impregnated consolidated warp knit fabric by wet coagulation to deposit the elastomeric polymer substantially uniformly throughout the thickness of said consolidated warp knit fabric.

2.  A method according to Claim 1 wherein removal of solvent from said impregnated consolidated warp knit fabric is carried out without forming a substantially continuous layer of elastomeric polymer on either of the first or second outer surfaces of the consolidated warp knit fabric.

3.  A method according to Claim 1 or Claim 2 wherein said elastomeric polymer is a polyurethane.

4.  A method according to any of Claims 1 to 3 wherein said solvent is selected from dimethylformamide and dimethylacetamide.

5.  A method according to any of Claims 1 to 4 wherein said elastomeric polymer is spandex.

6.  A method according to any of Claims 1 to 5 wherein said warp knit fabric comprises inelastic fibers selected from polyester and nylon.

7.  A method according to any of Claims 1 to 6 wherein said warp knit fabric has been produced on a Ketten, Raschel or tricot knitting machine.

8.  A method according to any of Claims 1 to 7 wherein a pigment has been added to said elastomeric polymer solution.

9.  A method according to any of Claims 1 to 8 which comprises an additional step of applying an antimicrobial finish to said impregnated consolidated warp knit fabric or of adding an antimicrobial agent to the elastomeric polymer solution used in fabric impregnation.

10. A method according to any of Claims 1 to 9 wherein said consolidated warp knit fabric is sanded on one or both sides prior to elastomeric solution impregnation.

**Patentansprüche**

1.  Verfahren zum Ausbilden eines dehnbaren elastischen Stoffs, wobei das Verfahren die Schritte umfasst:

    a) Bereitstellen eines Kettengewirks, das eine Dicke, eine Anfangsbreite, eine erste und eine zweite Außenfläche, eine Maschinenrichtung, eine Querrichtung und eine anfängliche flächenbezogene Masse von 15 bis 50 Gramm pro Quadratmeter aufweist;
    b) gleichmäßiges Ziehen des Kettengewirks durch Anlegen einer Kraft in Maschinenrichtung, um dadurch ein verfestigtes Kettengewirk mit einer Querrichtungsbreite, die 30 % bis 90 % seiner Anfangsbreite beträgt, bereitzustellen;
    c) im Wesentlichen gleichförmiges Imprägnieren des verfestigten Kettengewirks mit einer Lösung, die ein in einem Lösungsmittel gelöstes elastomeres Polymer umfasst; und
    d) Entfernen des Lösungsmittels aus dem imprägnierten verfestigten Kettengewirk durch Nasskoagulation, um das elastomere Polymer im Wesentlichen gleichförmig über die gesamte Dicke des verfestigten Kettengewirks abzuscheiden.

2.  Verfahren nach Anspruch 1, wobei das Entfernen des Lösungsmittels aus dem imprägnierten konsolidierten Kettengewirk ausgeführt wird, ohne dass eine im Wesentlichen durchgängige Schicht von elastomerem Polymer auf sowohl der ersten als auch der zweiten Außenfläche des verfestigten Kettengewirks gebildet wird.

3. Verfahren nach Anspruch 1 oder Anspruch 2, wobei das elastomere Polymer ein Polyurethan ist.

4. Verfahren nach einem der Ansprüche 1 bis 3, wobei das Lösungsmittel aus Dimethylformamid und Dimethylacetamid gewählt ist.

5. Verfahren nach einem der Ansprüche 1 bis 4, wobei das elastomere Polymer Spandex (Elastan) ist.

6. Verfahren nach einem der Ansprüche 1 bis 5, wobei das Kettengewirk unelastische Fasern umfasst, die aus Polyester und Nylon gewählt sind.

7. Verfahren nach einem der Ansprüche 1 bis 6, wobei das Kettengewirk auf einer Ketten-, Raschel- oder Trikot-Kettenwirkmaschine hergestellt wurde.

8. Verfahren nach einem der Ansprüche 1 bis 7, wobei der elastomeren Polymerlösung ein Pigment zugegeben wurde.

9. Verfahren nach einem der Ansprüche 1 bis 8, das einen zusätzlichen Schritt umfasst: Auftragen einer antimikrobiellen Appretur auf das imprägnierte verfestigte Kettengewirk oder Zugeben eines antimikrobiellen Mittels zu der bei der Stoffimprägnierung verwendeten elastomeren Polymerlösung.

10. Verfahren nach einem der Ansprüche 1 bis 9, wobei das verfestigte Kettengewirk vor der Imprägnierung mit der elastomeren Lösung auf einer oder beiden Seiten angeraut wird.

**Revendications**

1. Procédé de formation d'un tissu élastique étirable, ledit procédé comprenant les étapes suivantes:

   a) prévoir un tricot à chaîne présentant une épaisseur, une largeur initiale, des première et seconde surfaces extérieures, un sens machine, une direction transversale et un poids de base initial de 15 grammes à 50 grammes par mètre carré;
   b) étirer uniformément ledit tricot à chaîne en appliquant une force dans le sens machine afin de former ainsi un tricot à chaîne consolidé qui présente une largeur dans la direction transversale qui correspond à entre 30 % et 90 % de sa largeur initiale;
   c) imprégner sensiblement uniformément ledit tricot à chaîne consolidé avec une solution contenant un polymère élastomère dissous dans un solvant; et
   d) éliminer le solvant dudit tricot à chaîne consolidé imprégné par coagulation humide afin de déposer le polymère élastomère sensiblement uniformément sur la totalité de l'épaisseur dudit tricot à chaîne consolidé.

2. Procédé selon la revendication 1, dans lequel l'élimination du solvant dudit tricot à chaîne consolidé imprégné est exécuté sans former de couche sensiblement continue de polymère élastomère sur l'une ou l'autre des première ou seconde surface extérieurs du tricot chaîne consolidé.

3. Procédé selon la revendication 1 ou la revendication 2, dans lequel ledit polymère élastomère est un polyuréthane.

4. Procédé selon l'une quelconque des revendications 1 à 3, dans lequel ledit solvant est sélectionné parmi le diméthylformamide et le diméthylacétamide.

5. Procédé selon l'une quelconque des revendications 1 à 4, dans lequel ledit polymère élastomère est l'élasthanne.

6. Procédé selon l'une quelconque des revendications 1 à 5, dans lequel ledit tricot à chaîne comprend des fibres inélastiques sélectionnées parmi le polyester et le nylon.

7. Procédé selon l'une quelconque des revendications 1 à 6, dans lequel ledit tricot à chaîne a été produit sur un métier Ketten, Raschel ou à tricoter.

8. Procédé selon l'une quelconque des revendications 1 à 7, dans lequel a pigment a été ajouté à ladite solution de polymère élastomère.

9. Procédé selon l'une quelconque des revendications 1 à 8, comprenant une étape supplémentaire qui consiste à appliquer une finition antimicrobienne audit tricot à chaîne consolidé imprégné ou à ajouter un agent antimicrobien à la solution polymère élastomère utilisée pour l'imprégnation du tissu.

10. Procédé selon l'une quelconque des revendications 1 à 9, dans lequel ledit tricot à chaîne consolidé est poncé sur un coté ou sur les deux côtés avant l'imprégnation avec la solution élastomère.

**REFERENCES CITED IN THE DESCRIPTION**

**Patent documents cited in the description**

- US 4103485 A
- WO 0029654 A
- WO 04022827 A
- US 4366814 A
- US 5910224 A
- US 6942896 B
- WO 8301736 A
- WO 9737073 A
- US 4487040 A
- US 4802346 A
- US 6122940 A
- WO 88020038 A
- WO 03023105 A